# EUROPEAN PATENT APPLICATION

(11) **EP 0 631 767 A1**
(43) Date of publication of application: **04.01.1995**
(21) Application number: 94110176.8
(22) Date of filing: 30.06.1994
(51) Int. Cl.: A61F 13/15

(54) **Sanitary articles**

(30) Priority: 03.07.1993 GB 9313812; 16.05.1994 GB 9411303
(71) Applicant: BREGER GIBSON LIMITED, Holywell, Clwyd CH8 7HJ (GB)
(72) Inventor: Shipley, Arthur Roger, Clwyd LL19 9PU (GB)
(74) Representative: Dummett, Thomas Ian Peter

(57) **Abstract**

The present invention relates to a disposable sanitary article, notably a diaper, formed from a composite fabric having a fluid impervious outer layer, an intermediate absorbent layer and an inner next-to-the-skin layer, characterised in that an axially elongated slit is formed in a first sheet in the next-to-the-skin layer of the fabric, preferably extending over the crotch area of the diaper, and in that the opposed axial lips of the slit are caused to adopt an inwardly directed and upstanding configuration, for example by elastication along the edges of the lips, and thereby provide a pair of opposed axial barrier cuff walls.

The invention also provides a method for making such an article.

## Description

The present invention relates to disposable sanitary articles, notably to a disposable diaper having upstanding cuffs to retain bodily fluids and/or solids excreted into the diaper by a wearer.

### BACKGROUND TO THE INVENTION:

Disposable diapers typically comprise a fluid permeable next-to-the-skin layer and a fluid impervious outer layer with an intermediate fluid absorbent pad or layer.

The next-to-the-skin, or inner, layer is typically made from one or more sheets of a hydrophilic woven or non-woven material, for example a polypropylene or other resin sheet or a cellulosic tissue or other fluid permeable sheet made from randomly orientated fibres. The layer may have several such sheets laid one upon the other to vary the properties of the individual sheets. The term next-to-the-skin layer is therefore used herein to denote a layer of the composite diaper fabric which is located between the skin of the wearer and the absorbent pad or layer of the diaper construction. The next-to-the-skin layer functions to allow the bodily fluids to pass through the sheet so that they can be absorbed by the intermediate absorbent pad or layer.

Various modification of such a next-to-the-skin layer have been proposed. These include treatment of part of the layer to form hydrophobic lateral portions and treatment to render the layer resistant to back flow of fluid once this has passed through the layer, so as to reduce re-wetting of the skin of a wearer. It has also been proposed the apply a coating of very fine fibres to the inner face of the layer to improve the hydrophilic nature of the layer and to aid transport of fluid through and away from the layer.

The outer layer is a water impervious sheet, for example one or more sheets of a polyethylene or other polymer, and serves to retain fluid within the diaper. Again, modifications to such a layer have been proposed, for example to perforate the outermost sheet or otherwise permit the passage of vapour but not fluid, so that at least part of the fluid carried by the absorbent pad or layer can be lost by evaporation through the outer layer without the outer face of the outer layer becoming damp to the touch.

The intermediate absorbent pad or layer is typically formed from a pad of cellulosic or other fibres, which may be solid or hollow, and serves to absorb fluid passing through the next-to-the-skin layer. Such a pad can be formed by fluffing up a layer of cellulosic fibres mechanically; by bonding together short lengths of fibres by heat, pressure or other techniques; or by forming a tangled mass of a long fibre, which may carry one or more heat, pressure or other forms of adhesive so that the fibre pad forms a coherent body. The pad can be formed as a single or multi-layer construction. The pad can be contained within an envelope of a tissue paper or other sheet material to retain the fibres as a pad of the desired shape. Such an envelope can be heat bonded or otherwise adhered to the surface layers of the fibres in the pad.

Typically, such a diaper is formed from its component parts as a length of a composite fabric from which the desired shape of diaper is cut by stamping, water jet or air blade cutters. If desired, the various layers making up the composite diaper fabric can be bonded together during the bringing together of the layers of the materials. This can be done, for example, by using a pressure or thermal adhesive or a UV set-able adhesive applied as a series of stripes or as an overall spray of the adhesive to the relevant faces of the layers. The cutting away of the parts of the diaper to give the desired anatomically shaped, ie. waisted shape, diaper can be done as each layer is introduced during the construction of the diaper. The cutting operation can also cause thermal bonding of the cut edges, as when a cutter/sealer bar is used to cut layers made from or adhered together by a thermoplastic resin, so as to form a sealed edge to the final diaper construction.

Typically, such diapers have a T or H plan shape with the upright of the T or the cross bar of the H passing between the legs of a wearer to lie adjacent the crotch area of the body of the wearer. This area of the diaper is typically formed with a waisted plan shape to assist the diaper to conform to the anatomical shape of the body of the wearer. The cross piece of the T or the uprights of the H are then passed around the waist of the wearer and the overlapping free ends thereof secured together to form a waistband around the body of the wearer and thus secure the diaper in position upon the body of the wearer. Preferably, the free ends of the T or H are secured together by means of a self adhesive or hook and eye type material pad or the like attached to the free ends or carried by elasticated straps carried terminally upon those free ends.

To assist a close fit of the diaper and to accommodate variations in the size of the wearer's body, it has been proposed to incorporate elastication into the waistband and/or axially along at least the crotch/anal region of the diaper so as to form transverse and/or longitudinal gasket cuffs along the edges of the diaper which reduce lateral seepage of urine and faeces between the diaper and the body of the wearer. Such elastication can be introduced by inserting tensioned elastic threads or tapes between two or more of the layers of the diaper fabric at the desired position during manufacture of the diaper; or by accordion folding the area to be elasticated and inserting or adhering an elastic thread in a relaxed state to achieve a shirred effect. Alternatively, a thermally elasticated thread, tape or strip can be applied in its elongated state to one face of the diaper or inserted between layers during manufacture of the composite fabric. Upon heating, such a material will revert to its shorter elastic state and achieve a similar shirred effect.

In order to further enhance the fit of such a diaper upon the body of a wearer, it has been proposed to incorporate two or more upstanding flexible barrier cuffs, one lying along each side of the crotch area of the diaper, which engage the body of the wearer to form a barrier wall along each side of the crotch area. These barrier cuffs are located laterally inwardly of the gasket cuffs and are usually present in addition to the gasket cuffs. Such barrier cuffs act to restrain the escape of fluid and faeces from the diaper, either by acting as a dam or by acting as a filter so that undesirable leakage of bodily fluids and/or solids is reduced as the diaper is flexed by the body movements of a wearer.

Typically, such barrier cuffs are provided as folds in the next-to-the-skin sheet of material or by extra axial strips of material at or adjacent the longitudinal edges of the crotch area of the diaper. The barrier cuffs are caused to be upstanding by carrying elastication along their free, or distal, longitudinal edges. Such elastication can be introduced by inserting a tensioned elastic thread into a sleeve formed along the distal edge of the fold or strip, by adhering such a tensioned thread at or adjacent the distal edge of the cuff or by applying a heat elasticisable thread tape or strip along the distal edge of the cuff.

The barrier cuffs are typically formed by folding the material of the next-to-the-skin layer to form an upstanding longitudinal wall of the material or by applying two parallel continuous lengths of material of the required width to the next-to-the-skin layer on its next-to-the-skin face; and securing the folds or extra strips in position by adhesive, or by thermal and/or pressure, UV or HF bonding along at least their laterally outward edges, leaving the laterally inward edges free. Where strips of additional material are applied to the next-to-the-skin layer to form the barrier cuffs, the strips can be of any suitable material, for example a hydrophobic form of the inner layer material or a fluid impervious material as used in the outer layer of the diaper. Typically, the whole transverse width of these folds or additional strips will be secured to the inner layer material at the waistband end areas of the diaper to form closed ends to the pocket created by the upstanding barrier cuff walls.

The number and location of the elasticated areas along the edges of the barrier and gasket cuffs, along the edges of the crotch areas and waistbands of the diaper can vary. For example, it will be usual to use three or more parallel rows of elastic threads along or inset from each of the lateral edges of the diaper in the crotch area. The barrier cuffs can be formed or secured laterally inwardly or outwardly of some or all of these rows of thread; and the thread or threads along the distal edge of the barrier cuff can be located at the distal edge of the fold or strip forming the cuff wall or can be inset some distance therefrom to give a ruffled type of edge to the barrier.

When the individual diapers are cut from the length of composite fabric, the tension in the fabric is released and the diaper adopts a concertina configuration due to the elastic threads, tapes or strips within its structure. This causes the barrier cuffs to upstand generally normal to the plane of the remainder of the diaper.

For convenience, the term disposable diaper will be used herein to denote in general the above basic type of diaper and its various modifications.

If care is not exercised during manufacture of such diapers, adhesive may be applied along part or all of the barrier cuff and prevent the barrier cuff wall from adopting an upstanding configuration. Furthermore, it is necessary to ensure accurate registration of the various components of the composite diaper fabric to form the gasket and barrier cuffs at the correct locations in the final diaper product. Where a barrier cuff is formed along the whole axial front to back length of the diaper, the barrier cuff has a tendency to flop over indiscriminantly inwardly or outwardly or to become twisted when the diaper is being applied to the wearer and this causes a source of leakage at the points where the cuff flops first inwardly then outwardly.

In the form of the barrier cuffs described in our co-pending PCT Application No GB92/01584, the cuffs are formed by cutting two or more parallel slits in the next-to-the-skin layer of the composite diaper fabric and raising the laterally outward lips of the slits by suitable elastication to form one upstanding cuff wall along the outer edge of each of the slits. This form of construction for the barrier cuffs reduces the above problems, does not require the use of extra material to form the barrier cuffs, and reduces material costs and the number of operations required to form the barrier cuffs.

In all the above forms of diaper it has been considered necessary to have the barrier cuffs located to either side of the absorbent pad in the crotch area of the diaper. Surprisingly, we have now found that a satisfactory pair of barrier cuffs can be formed by cutting a single slit along substantially the centre line of the next-to-the-skin layer of the diaper and allowing the lips of the slit to be made to upstand, each lip then forming an upstanding barrier cuff wall. Furthermore, by allowing the slit to gape there can be formed an aperture in the next the skin layer of the composite diaper fabric which assists rapid transfer of the urine or faeces onto the underlying absorbent pad. Since the aperture can be formed at any desired axial position on the diaper, the aperture can be located at the optimum position to receive the discharge from a male or female wearer of the diaper and thus render the diaper gender specific by simple positioning of the slit. Moreover, since the slit is formed in a single sheet of material, problems in securing accurate registration of additional strips of material to form the barrier cuffs is avoided.

The use of a single slit thus provides a simple and effective means for forming both barrier cuffs and of forming an aperture which aids rapid assimilation of the discharge from the wearer and of enabling gender specific diapers to be made without the need for any additional manufacturing operations or additional features in the diaper design, thus simplifying manufacture and reducing costs.

It has been proposed in British Patent Application No 2265834 A (published 13 October 1993) to cut a single axial slit in the next-to-the-skin layer of a diaper with terminal transverse cuts at each end of the axial slit, so as to form two axial opposed flaps of material. The flaps are folded outwardly from the line of the slit to form an axial aperture. The flaps are elasticated along the fold line and at the outwardly directed distal edge of the flap to form a surface which is orientated generally parallel to the plane of the next-to-the-skin layer. This surface bears against the body of a wearer of the diaper to provide a seal against lateral escape of fluid and faeces. By virtue of the outward direction of the folding of the flaps, the surface adopts a slightly upstanding configuration and may have a tendency to become entangled with the genital skin of the wearer, which can cause discomfort.

If the transverse cuts extend sufficient distance transversely to enable the flaps to be formed sufficiently far away from the centre line of the diaper that they do not contact the genital areas of the wearer, the amount of material which must then be folded to form the flaps causes difficulties in handling and forming the folds and the surface, leading to errors in manufacture.

Surprisingly, we have found that if the flaps are formed so that they are directed inwardly, as opposed to outwardly, entanglement with the genital skin is reduced and the need to form the flaps laterally outwardly of the genital skin area is avoided. The amount of material which has to be handled during formation of the flaps can be further reduced by forming the aperture by cutting away part of the material of the next-to-the-skin layer and manufacture of the diaper is simplified. Furthermore, since the material forming the flaps lies substantially co-planar with the next-to-the-skin layer of the composite diaper fabric until the tension in the elastication in the flaps and other components of the diaper is released at the end of manufacture of the diaper, it is possible to employ a simple and effective means for forming an aperture in that layer and forming the elasticated edge to the flaps, which further simplifies manufacture of the diaper.

### SUMMARY OF THE INVENTION:

Accordingly, the present invention relates to a disposable sanitary article, notably a disposable diaper, formed from a composite fabric comprising a fluid permeable next-to-the-skin layer, an outer fluid impermeable layer and an intermediate fluid absorbent layer or pad and having an axially elongated slit or aperture formed in a sheet of the next-to-the-skin layer of the composite fabric to provide an apertured sheet, the aperture or slit extending along or adjacent the centre line of the article, characterised in that:
a. the aperture or slit having opposed axial lips is formed in a single sheet of the composite diaper fabric within the plan area of the underlying absorbent pad or layer;
b. the opposed axial lips of the aperture or slit are provided with means, notably elastication, for raising the distal edges of the lips with respect to the plane of the apertured sheet; and
c. the distal edges of the lips are caused to adopt an inwardly directed and upstanding configuration with respect to the aperture or slit so as to provide a pair of opposed inwardly directed upstanding axially extending barrier cuffs in the next-to-the-skin face of the article.

Preferably, the slit is caused to gape transversely by a significant amount or is formed with a significant transverse dimension so as to provide an aperture in the next-to-the-skin layer of the composite fabric which assists transport of the discharge from a wearer to the underlying absorbent layer or layers of the article. It is also preferred that the axial location of the aperture or slit be selected according to the gender of the intended wearer of the article so as to locate the aperture or slit in register with the source of the discharge of bodily fluids from the wearer.

In a preferred embodiment, there is provided a secondary sheet of material underlying at least the plan area of the aperture or slit in the apertured sheet, such secondary sheet of material being located intermediate the apertured sheet and the absorbent pad or layer. Preferably, there is provided a third sheet intermediate said secondary sheet and the absorbent layer or pad, said third sheet carrying one or more materials to enhance the absorption of fluid by the sheet, and preferably also having an aperture located at least in part in register with the plan area of the aperture or slit in the apertured sheet.

In a further preferred embodiment, the absorbent pad or layer is formed with a recess located at least in part in register with the plan area of the aperture or slit in the apertured sheet, which recess is adapted to receive and retain faecal solids deposited on the diaper by a wearer.

Other disposable sanitary articles, for example incontinence pads or sanitary towels, have a similar construction and carry one or more barrier or gasket cuffs. The invention can also be applied to such other articles. However, for convenience, the invention will be described hereinafter in terms of a disposable diaper.

The invention also provides a method for forming a pair of barrier cuffs in a disposable sanitary article formed from a composite fabric comprising a fluid permeable next-to-the-skin layer, an outer fluid impermeable layer and an intermediate fluid absorbent layer or pad, characterised in that the method comprises:
a. forming an axially elongated slit or aperture in a single sheet in the next-to-the-skin layer of the fabric to provide an apertured sheet, the aperture or slit having axial opposed lips along each axial edge thereof, the aperture or slit extending along or adjacent the centre line of the article and being located within the plan area of the underlying absorbent pad or layer;
b. providing the opposed axial lips of the aperture or slit with means for raising the distal edges of the lips with respect to the plane of the apertured sheet so as to provide a pair of opposed inwardly directed upstanding axially extending barrier cuffs in the next-to-the-skin layer of the article when the article is in its relaxed state.

Whilst the article will normally be produced in a lay flat state in which the tension within the article is not released, for example due to the article being packed in a restraining packaging, release of the article prior to use will allow the elastication or other means to cause the distal edges of the lips along the aperture or slit to adopt their upstanding configuration. For convenience, the invention will be described hereinafter in terms of the article as ready for use.

In a particularly preferred aspect of the method of the invention, the next-to-the-skin sheet in which the slit is cut is formed from a non-woven fibrous material, and the slit is formed as an aperture in the sheet by removing material from the sheet, characterised in that:
a. the fibrous material of the next-to-the-skin sheet is partially cut through along the periphery of the intended aperture, whereby the material of the sheet within the intended aperture is removably retained within the sheet; and
b. the material of the sheet within the intended aperture is subsequently removed, preferably by applying suction to the material and separating it from the remainder of the sheet to form the aperture in the sheet, notably during or after the bringing together of the components of the composite fabric from which a disposable sanitary article is to be formed.

Preferably, the aperture or slit is formed by cutting the exposed sheet of the next-to-the-skin layer of the diaper composite fabric. However, it is within the scope of the present invention to form the aperture or slit in a sheet of material underlying the exposed next-to-the-skin sheet where the exposed sheet can flex sufficiently to allow the distal edges of the lips of the aperture or slit formed in the underlying sheet to raise the overlying sheet to form the barrier cuffs which can conform to the body shape of a wearer of the diaper.

For convenience, the invention will be described hereinafter in terms of a diaper having the aperture or slit formed in the exposed next-to-the-skin sheet of the composite fabric.

It will appreciated that the aperture or slit can be formed transversely adjacent either or both axial ends of the diaper, as well as or alternatively to being formed axially, so as to provide transverse barrier cuffs adjacent the end(s) of the diaper. However, the invention will be described hereinafter in terms of an aperture or slit which is formed axially in the diaper to provide a pair of opposed inwardly directed axial barrier cuffs.

The aperture or slit is located within the plan area of the underlying absorbent pad or layer. However, it is preferred that the aperture or slit extend axially only over the area of the diaper into which the bulk of the bodily fluids and solids are to be discharged by a wearer. Thus, for diapers intended for use upon a male baby or person, the slit will extend axially over the anal area and over an area extending axially forward thereof into that length of the diaper which is to be folded upward over the lower abdomen of the wearer. In the case of a diaper to be worn by a female, the slit need not extend axially forward as far from the anal area and will usually extend rearwardly of the anal area. As indicated above, gender specific diapers can readily be fabricated merely by varying the location and/or length of the slit. In each case, the slit will extend for from 25 to 75% of the axial length of the diaper and does not extend into the waistband area of the diaper.

As indicated above, the aperture or slit is located substantially along the centre line of the diaper so that two opposed axial lips are formed lying symmetrically within the plan area of the absorbent pad or layer of the diaper. However, it may be desired to form the aperture or slit off-centre and/or at an angle to the centre line of the diaper, for example as a diagonal slit extending to either side of the centre line of the diaper. For convenience, the invention will be described hereinafter in terms of an aperture or slit which is formed substantially along the centre line of the diaper.

As stated above, the slit is preferably allowed to gape to a significant extent so as to provide an aperture with a transverse dimension at its maximum gape of at least 1 to 2 cms when worn by a wearer. This can be achieved due to the axial length of the slit and the transverse tensions applied to the slit during use. To achieve such a gape, the apertured sheet is not adhered to the underlying material of the diaper construction over the intended plan area of the aperture. At least part of the gape of the slit is also caused by means of the type and configuration of the elastication or other means for raising the distal edges of the lips.

In a particularly preferred form of the invention, the axial slit connects with a terminal transverse cut at each end thereof so that the opposed axial lips of the axial slit are formed as axial flaps of material which can be folded back to form the aperture in the sheet and to provide the opposed axial barrier cuff walls. Preferably, the free axial distal edges of the flaps are folded over to form an axial sleeve along each flap within which tensioning elastication is located as described below. Preferably, the edge of the flap is folded downwardly and outwardly under the remaining material of the apertured sheet so that the distal edge of the flap presents a smooth rounded edge to the skin of the wearer rather than a cut edge as occurs when the edge of the flap is folded upwardly then outwardly leaving the cut edge of the flap exposed on the upper face of the flap.

The opposed axial lips of the slit are provided with axial elastication or other means whereby the distal edges of the lips are caused to upstand from the surrounding surface of the apertured sheet. Thus, the fabric of the sheet into which the slit is cut can be provided with an underlying secondary sheet or other means which has a set or bow imparted to it which causes the distal edges of the lips of the slit to curl upwardly. Alternatively, the slit can be cut in a sheet of material which is stretched transversely to the axis of the diaper so that the cutting of the slit releases the restraint on the fabric and allows the distal edges of the lips of the slit to curl upwards.

However, it is preferred to cause the distal edges of the lips of the slit to upstand by foreshortening the lip axially by means of one or more elasticated threads of the type conventionally used in fabricating a diaper. The axial foreshortening of the lips by the elastication also causes them to retain their inwardly directed orientation and reduces the risk that the lips will flop over to be directed outwardly during use. The elastication is typically provided by one or more elastic threads applied under tension using conventional techniques, for example by applying a thread coated with a hot melt or pressure sensitive adhesive axially along the desired area of the lip and applying heat and/or pressure thereto, for example as the next-to-the-skin layer is bonded to the underlying portions of the diaper fabric. Alternatively, the lip of the slit can be folded or rolled transversely to form an axial sleeve around the thread and the sleeve secured upon the thread by adhesive or the like.

The elastication can be provided by one or more of the elastic threads already present in the composite diaper fabric, or can be incorporated as extra threads using any suitable technique.

In order to ensure that the lips or the flaps formed thereat adopt an upstanding configuration and are directed inwardly as opposed to outwardly, the elastication can extend axially beyond the extremities of the slit and into the unslit area of the next-to-the-skin layer, for example by incorporating one or more threads of elastic which run for substantially the whole axial length of the diaper into the distal edges of the flaps. Such elastication also ensures that the axial ends of the flaps are substantially co-planar with the remainder of the apertured sheet and thus form ends to the pouch or pocket formed by the upstanding barrier cuff walls. However, we have found that axial elastication within the length of the flaps will usually provide sufficient inward bias to prevent the resultant barrier cuff walls from flopping outward during use.

As indicated above, the aperture preferably has a transverse width of at least 1 cm or more. This can be achieved by folding back the opposed axial distal edges of the flaps as described above. Alternatively, part of the material of the sheet can be removed, as when a slot having an appreciable transverse dimension is formed. This has the advantage that the amount of material which is to be incorporated into the flaps along each axial lip of the aperture is reduced, thus facilitating formation and handling of the flaps.

Typically, it will be desired to form the aperture with a transverse dimension of from 25 to 100, preferably 40 to 75, mms. The axial aperture will be formed to have an initial transverse dimension of from 40 to 90% of the desired transverse width, and the flaps along each axial edge of the aperture are folded downwardly and under the remainder of the material to extend the axial slit to the desired transverse dimension of the aperture.

Where material is to be removed from the sheet to form the aperture, it is preferred only partially to cut through the material of the sheet so as to retain the material which is to be removed and form the aperture within the web of the sheet. This permits the sheet to be transported as a whole until such time as it has been brought into register with some or all of the other components of the composite fabric from which the diaper is to be formed. The material within the intended location of the aperture can then be simply removed by a suction pad or the like. By retaining the material to be removed in the sheet, the sheet can be readily handled using conventional machinery with less risk of distortion and resultant difficulties in securing correct alignment and registration with other components than when a sheet with preformed apertures is used. Typically, the sheet is partially cut with a suitable cutter blade whose pressure of application is adjusted so that the blade does not penetrate through the final 1 to 5% of the thickness of the material of the sheet. Preferably, this partial cut extends continuously for the full length of the periphery of the intended aperture. However, the partial cut may extend intermittently around the periphery with cuts through the full thickness of the material extending between the partially cut portions.

As indicated above, the partial cut enables the sheet to be handled using conventional machines, but allows ready removal of the material within the partial cut periphery to be removed by a suction pad or other means. The optimum extent of the depth and/or peripheral extent of the partial cut will depend upon the sheet handling mechanism and the suction pad or other mechanism for removing the material which are being user by an operator and can readily be determined by simple trial and error tests.

It is also preferred that the axial extremities of the aperture and the opposed axial barrier cuff walls form a pouch or pocket which retains fluids and solids against axial movement. As indicated above, this may be achieved by extending the elastication of the distal edges of the lips of the aperture beyond the axial extremities of the aperture or slit. Preferably, the aperture or slit terminates within the exposed axial length of the apertured sheet which is not adhered or otherwise bonded to the underlying material of the diaper construction. The elastication of the lips along the slit causes the material of the apertured sheet to be raised with respect to the underlying material of the diaper and thus form a pouch extending under the apertured sheet beyond the plan area of the aperture.

The proximal bases of the flaps along the slit are preferably located transversely inwardly of the edges of the plan area of the underlying absorbent layer or pad in the diaper construction. The absorbent pad is thus exposed through the aperture or slit in the apertured sheet and the barrier cuff walls inhibit the lateral flow of material away from that exposed area of the absorbent pad before it can be absorbed by the pad.

Thus, in a particularly preferred form of the invention, the diaper has a conventional composite material construction with a single aperture or slit in the next-to-the-skin sheet located substantially along the centre line of the diaper, and the aperture formed by the gaping slit or by the folding back of the distal edges of the flaps along the aperture or slit has a maximum transverse dimension of from 2 to 7.5 cms lying wholly within the plan area of the absorbent layer of the diaper.

The slit(s) can be formed by any suitable technique, for example by cutting a curved, dog leg or other shaped slit or aperture in the next-to-the-skin layer of material as it is fed to the diaper fabric construction process. If desired, the slit can be formed by removing part of the plan area of the sheet so that the slit has a significant transverse dimension, preferably using the partial cut technique described above. The slit(s) or partial cuts can be formed with a knife blade, a water jet or air jet cutter, or with a rotary die cutter as conventionally used in cutting diaper fabric. In the case of a thermoplastic sheet material, the slit can be cut and the tensioned elastic thread or other lip raising means secured in position by the use of a heated cutter/sealer.

If desired, further slits lying to either side of the central slit of the invention can be formed as described in our copending PCT Application No GB92/01584, so that a plurality of parallel barrier cuffs are formed lying symmetrically or asymmetrically to each side of the axial centre slit of the diaper. The axial slit can be combined with transverse slits to provide both axial and transverse cuffs. However, it will usually be sufficient to form one axial slit along the centre line of the diaper to form a pair of leg cuffs, the additional seals usually present in diapers being provided at the lateral edges of the diaper by conventional elasticated gasket cuffs and for convenience the invention will be described herein in terms of the formation of a single pair of barrier cuffs from a single axial slit.

In a preferred aspect of the invention, the next-to-the-skin sheet of material, notably a spun bonded or thermally bonded non-woven polyester or polypropylene fibre or a cellulosic fibre fabric sheet, is provided with at least part of the elastication of the crotch area of the diaper attached thereto as a series of parallel axial threads over an area which extends across substantially the width of the diaper. The threads are secured to the material over their axial length extending into the axial area where the aperture or slit is to be formed. Preferably, the threads are not secured to the material for between 25% and 85% of the axial length of the area within which the aperture or slit is to be formed. One or more of the axial elasticated threads are located in register with or adjacent to where each of the axial distal edges of the aperture or slit are to be formed. The axial aperture or slit, preferably with a transverse cross-cut at each end thereof to form the axial flaps lying to each side of the aperture or slit, is cut in the sheet. The cross-cuts also sever the elasticated thread(s) within the area of the slit or aperture. However, these are secured to the material of the flaps at the axially terminal portions thereof so that tension is retained in the thread(s) and the flaps incorporating them. Alternatively, no elastic threads may be retained in the material of the flaps, and the elastication of the edges of the aperture or slit is achieved by virtue of the elastic thread(s) running alongside but outwardly of the aperture or slit as cut. In this case the elastic thread(s) extend beyond the axial extremities of the aperture and the thread(s) are retained in a tensioned state. The flaps of material are folded around the elastic thread(s) underlying them or running adjacent them to form sleeves around the thread(s). Preferably from 40% to 60% of the axial length of the thread(s) within the sleeve is not adhered to the sleeve material and is free to move axially within the sleeve. Thus, when axial tension is released from the diaper, for example when the individual diaper is cut from the composite fabric, the elasticated thread causes the sleeve to contract axially with respect to the remainder of the diaper so as to cause the distal edges of the flaps to rise and form the upstanding barrier cuff walls to either side of the aperture or slit.

The axial slit and the resultant barrier cuff walls are formed in a single sheet of material (the apertured sheet) which can form the exposed next-to-the-skin sheet, and the gape of the aperture or slit gives direct access to the underlying absorbent layer or pad. However, the aperture or slit can be formed in an extra sheet of material which is applied over what would have been the exposed next-to-the-skin sheet of the diaper so that an intermediate second sheet of material which has retained its integrity is located between the apertured sheet and the absorbent layer or pad of the diaper. Alternatively, the slit can be formed in the exposed next-to-the-skin sheet and the secondary sheet incorporated intermediate the apertured sheet and the absorbent layer. The presence of this secondary sheet is of particular application where the absorbent pad or layer is formed from a material which would be liable to escape through the aperture in the apertured sheet, for example where the absorbent layer is formed from a loose mass of fibres which are not bonded to one another or retained within an envelope or where the absorbent layer contains loose granules of a super absorbent additive to enhance the fluid absorbent properties of the diaper.

In a further alternative, an extra third sheet of material is provided intermediate the secondary sheet and the absorbent pad or layer, which third sheet can also be provided with an aperture. Preferably, the aperture in this third sheet is smaller than the aperture in the apertured sheet and is at least in part in register with the slit or aperture in the apertured sheet. Preferably, this third sheet carries particles of a super absorber bonded to its upper or lower faces. Such a third intermediate sheet can be made from a coherent high loft material or from a conventional woven or non-woven material.

Thus, the diaper of the invention can be readily formed by applying a suitably apertured and elasticated sheet as a top sheet upon or as an intermediate sheet within a conventional composite diaper fabric, thus reducing the amount of modification required to the diaper production process and machinery. The secondary sheet underlying the apertured sheet can extend for the full width and length of the diaper. However, it may be preferred that the secondary sheet be narrower than the apertured sheet on the grounds of economy, provided that the secondary sheet extend laterally and axially beyond the plan area of the axial slit or aperture. Surprisingly, we have found that the use of a secondary sheet underlying the apertured sheet and extending laterally to or adjacent the edge of the underlying absorbent pad or layer and secured, for example by an adhesive bead or a weld, to the underside of the apertured sheet also assists retention of the distal edges of the flaps formed in the apertured sheet in an inwardly directed orientation.

The underlying structure of the remainder of the diaper, that is the absorbent layer or pad and the fluid impervious outer layer, can be of conventional design and construction. However, it is particularly preferred that the next-to-the-skin layer and/or the absorbent layer carry and/or contain one or more additives which enhance its fluid absorption properties. Thus, it is preferred that the exposed next-to-the-skin sheet or the secondary non-woven sheet immediately underlying that apertured sheet carry adhered to the under, or absorbent-layer-adjacent, face thereof particles of a super absorbent material. Typical of such materials are cross-linked polyacrylate homo-or co-polymers, notably co-polymers between acrylic acid or acrylonitrile with vinylic monomers or cellulosic materials. Such materials preferably have a particle size of from 100 to 1000 micrometres and absorb up to 200 times their own weight of urine. If desired, such super absorbent particles, eg. as granules or powders, can also be incorporated into the absorbent layer itself, for example by applying a layer of such particles to an initial layer of the fibres used to form the absorbent layer and then applying the remainder of the fibres to complete the absorbent layer. A layer of super absorbent particles can also be applied to the next-to-the-skin face of the absorbent layer and bonded in place by thermal bonding or by the application of an adhesive. If desired, the particles and/or adherent layers can contain localised higher concentrations of the super absorber in the regions where maximum absorbency is required.

It is also preferred that the upper face of the absorbent pad or layers be formed with a recess which is at least in part in register with the slit or aperture in the apertured sheet and serves to receive and retain faecal solids deposited on the diaper by a wearer. The recess can be formed by cutting an appropriate aperture in a top layer of the absorbent pad and bonding that layer to an non-apertured basal layer, or by any other suitable technique.

As indicated above, it is preferred that the various layers of the diaper composite fabric be bonded together to form a unitary construction, for example by the application of heat and/or pressure or the use of adhesive coatings, notably UV of high frequency radiation cured adhesives. The bonding together of the various layers assists retention of the particles of super absorber in the desired locations. However, it is preferred that the apertured sheet is not adhered or bonded to the underlying sheets of the composite fabric over at least the plan area of the aperture formed in the apertured sheet to aid the formation of the upstanding barrier cuff walls when the tension is released from the diaper. This will also form pockets between the apertured sheet and the underlying sheet which retain material in the region of the slit or aperture and hence of the recess in the absorbent pad or layer.

The invention has been described above in terms of a disposable diaper. However, the invention can be applied to other disposable sanitary articles which are to be applied to or worn by a person to receive and absorb urine, blood or other bodily fluids, for example sanitary pads or incontinence pads, or to pads which are to be inserted into panty hose or other apparel for disposal separately; and to wound dressings or adhesive plasters which are applied to the person. For convenience, the term disposable sanitary articles will be used herein to denote in general all disposable articles which are to be worn upon or applied to the person to receive and absorb bodily fluids.

### DESCRIPTION OF THE DRAWINGS:

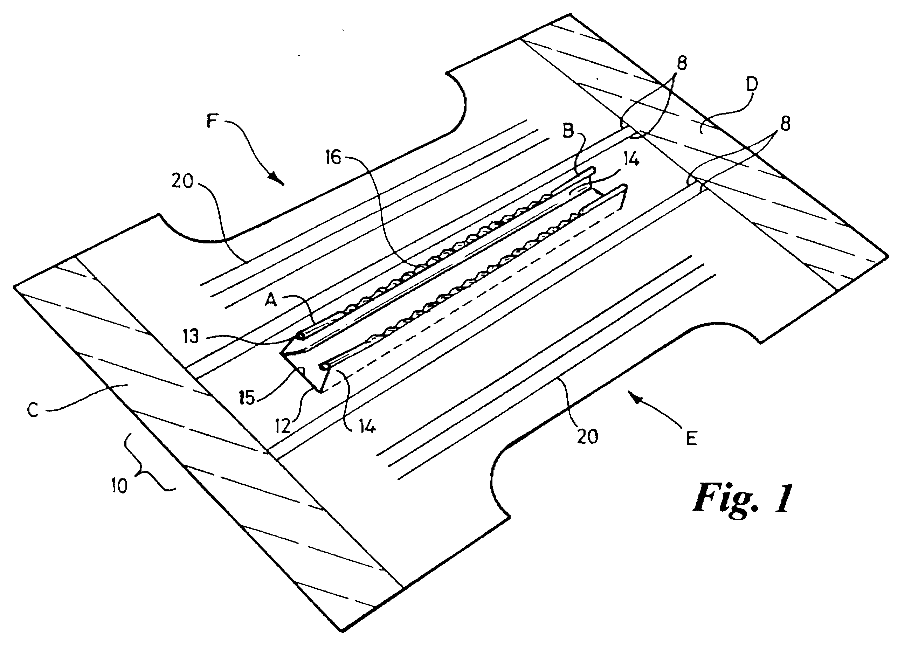

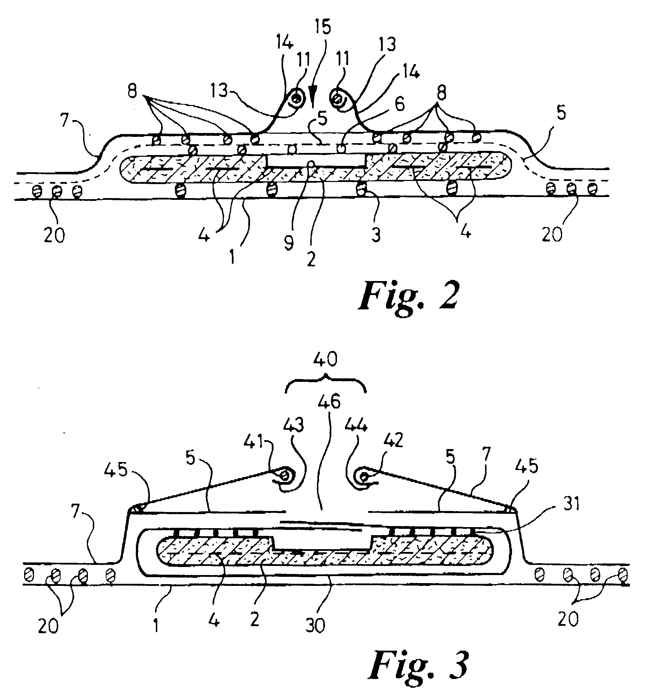

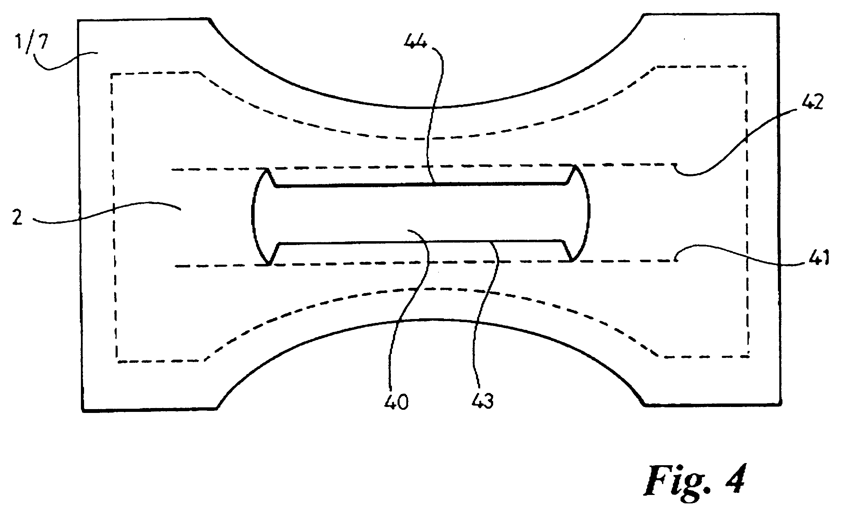

To aid understanding of the invention, a preferred form thereof will now be described by way of illustration with respect to the accompanying drawings, in which Figure 1 is a perspective view from the next-to-the-skin side of the diaper; Figure 2 is a transverse cross-section through the diaper of Figure 1; Figure 3 is a transverse cross-sectional view of an alternative form of the diaper of Figure 1 in which part of the next-to-the-skin sheet is removed to form the aperture; and Figure 4 shows the plan view of the apertured diaper of Figure 3 prior to folding over the flaps along each side of the aperture.

### DESCRIPTION OF THE PREFERRED EMBODIMENT:

The diaper comprises an outer fluid impervious sheet layer 1, for example an imperforate or microporous sheet of polyethylene or other water impervious plastic. To this is applied a pad 2 of cellulosic fibres, for example the loose bed of wood fibres obtained by passing a sheet of pulp board card through a hammer mill or the like. The pad can be contained within a tissue paper or similar envelope (shown in Figure 3 as item 30) and is preferably bonded to the outer layer 1 by axially extending beads 3 of a hot melt adhesive or a coating of a pressure sensitive adhesive applied over the interface area between sheet 1 and pad 2. Within pad 2 is formed a layer of polyacrylate super absorber granules 4 to enhance the fluid absorption properties of the pad 2. The upper face of pad 2 is provided with an axial recess 9 lying at least in part within the plan area of, ie. in register with, the aperture formed as described below in the next-the-skin sheet 7 of the diaper. This recess 9 can be formed by applying a suitably apertured upper portion sheet of absorbent material to an un-apertured basal sheet to form a composite pad 2. The materials used in the upper and basal portions of the pad may have different properties and the layer 4 of super absorber particles can be incorporated at the interface between the two portions of the pad.

The next-to-the-skin layer of the diaper composite construction is applied over pad 2. This layer typically comprises a first non-woven sheet 5, preferably of a polypropylene polymer or a cellulosic bonded fibre sheet, which is pervious to bodily fluids. This first sheet can be a conventional hydrophobic material, or can have the axially central area treated with a surfactant, or can be partially cut away to form to an aperture at least in part in register with the recess 9 in the underlying pad 2, to aid passage of urine and other fluids through the sheet. This sheet can extend laterally for the full width of the diaper as shown dotted in Figure 2, but preferably extends only over the plan area of the underlying absorbent pad 2 as shown in Figure 3. Sheet 5 underlies the axial aperture to be formed in the next-to-the-skin sheet 7 described below.

Where sheet 5 is provided with an aperture, a further sheet 5a (not shown) may be provided if desired which underlies or overlies at least the aperture in sheet 5 to prevent escape of fibres, particles of super absorber or other materials from the pad 2.

Bonded to the underside of the sheet 5 are a series of axial parallel elastic threads 6 which are fed to the interface between pad 2 and sheet 5 under tension during construction of the diaper. Preferably, the threads 6 are coated with a contact or hot melt adhesive so that passage of the diaper during its construction through the nip of a pressure and/or heated roller, preferably having a diamond or other shaped pattern of upstanding ribs on its surface, will cause the component layers of the diaper to bond to one another with the threads retained in position under tension. If desired, further particles of the super absorber can be carried on the upper and/or lower face of sheet 5.

As shown in Figure 2, the next-to-the-skin layer comprises a second sheet 7 of water pervious material carrying a series of tensioned axial elastic threads 8 applied over sheet 5 and bonded thereto. This second sheet extends the full width of the diaper to provide the exposed next-to-the-skin sheet of the diaper composite fabric construction. In order to permit the material of this sheet to form the axial upstanding barrier cuff walls as described below, the adhesive or other bonding agent attaching sheet 7 to sheet 5 is omitted over the area of the proposed position of the aperture in sheet 7. However, it is within the scope of the present invention, where pad 2 is sufficiently coherent, to dispense with sheet 7 and to form the required axial aperture described below in sheet 5, which will then extend for the full width of the diaper, or to dispense with sheet 5 and use only a single sheet 7 to form the next-to-the-skin layer of the composite diaper fabric.

A single axial aperture 10 is formed in sheet 7 substantially along the centre line of the diaper and extending axially over the area of the diaper to be in contact with the crotch area of the wearer. This aperture can be a slit having no appreciable transverse width when first formed or, more preferably, has a significant transverse dimension, for example 50 to 70 mms, when formed due to the removal of material from sheet 7. The aperture is preferably located between two of the elasticating threads 8 carried by the sheet 7 so that one of each of these two threads, hereinafter designated as threads 11, lies adjacent an axial lip of the aperture 10. If desired, transverse end cuts 12 can be formed in sheet 7 at each end of the axial slit 10 to aid formation of a flap of material to each side of the slit with the threads 11 located adjacent the free edges of the flaps. The free, or distal, axial edges of the flap can each be folded downwardly and inwardly around the adjacent thread 11 to form a sleeve 13 around the thread 11 and to create an axial rectangular aperture 15 in sheet 7. The threads 11 are retained in their tensioned state by being adhered to the material of the flaps over the terminal lengths A and B of the sleeves 13. The tension in threads 11 causes the sleeves 13 to contract axially as shown by the diagrammatic shirring effect 16 in Figure 1 and to cause the distal edges of the flaps to upstand from the plane of sheet 7 to form the barrier cuff walls 14 along each axial edge of the aperture 15 when the axial tension in the diaper is released when the individual diapers are cut from the length of composite fabric. The tension in the elastic threads 11 also causes the cuff wall 14 to be biassed inwardly towards the centre line of the aperture and not outwardly.

As shown in Figure 1, the barrier cuff walls can extend axially only over the crotch region of the diaper, that is over the length of the diaper over which they will need to be effective. This shorter length reduces the risk that the cuff will flop over when the diaper is applied to the wearer, which has hitherto represented a risk of negating the dam effect of the cuff.

The barrier cuffs are formed inwardly of the lateral edges of the underlying absorbent pad 2 so that material trapped by the cuff walls 14 is retained within the plan area of the absorbent pad 2 and solids are retained within recess 9 and do not escape laterally. Sheet 7 with the elastication and the slit 10 is preferably preformed and fed as such to the diaper construction line under tension as a substantially flat sheet.

The diaper can otherwise be of conventional design and construction with the layers of the diaper fabric bonded together, notably over the waistband areas C and D at each end of the diaper, and with the crotch areas E and F cut away to form an anatomically shaped diaper. Preferably, further elastication is provided at the edges of the diapers in the cut away crotch areas, for example by including further axial elastic threads 20 in these regions to aid formation of secondary gasket cuffs along the lateral edges of the diaper.

As shown, the slit 10 forms the aperture 15 in the sheet 7. Where sheet 7 is the sole sheet in the next-to-the-skin layer of the diaper, aperture 15 will provide a direct passage for urine and faeces to the absorbent pad 2. Pad 2 can be configured, and any super absorbent material 4 so distributed therein, to ensure that fluids flow rapidly away from the initial contact area with pad 2 into the bulk or reservoir portion of pad 2.

As shown in the alternative form of the diaper in Figure 3, the diaper comprises an outer water impermeable sheet 1, an absorbent fluff pad 2 containing particles of a super absorber 4. The pad 2 is wrapped in a tissue paper wrap 30 and further particles 31 of super absorber are applied to the upper face of pad 2. A next-to-the-skin sheet 7 is applied over pad 2 and extends to the periphery of the outer sheet 1 to form the overall composite structure of the diaper. The lateral edge of the diaper is elasticated with elastic threads 20 and the diaper is formed with waist bands C and D and cut outs E and F as with the diaper of Figure 1.

The sheet 7 is cut with an axial aperture 40 having the plan shape shown in Figure 4. It will be noted that the elastic threads 41/42 along each edge of the aperture 40 have not been severed as with the diaper of Figures 1 and 2, but remain intact and extend axially beyond the extremities of the aperture and into the uncut portion of sheet 7. Due to the shape of the aperture cut in sheet 7, a flap of material 43 and 44 is formed along each longitudinal edge of aperture 40. The aperture 40 will typically have an initial transverse width which is from 60 to 85% of the desired fully exposed width of the aperture so that the flaps 43 and 44 are comparatively thin and can readily be handled. The distal edges of these flaps are folded downwardly and laterally under the remainder of sheet 7 to form axial sleeves around the elastic threads 41 and 42. A secondary sheet 5 is located underlying sheet 7 and extends over the transverse width of pad 2. Sheet 5 is secured to the underside of sheet 7 by axial glue beads 45. Sheet 5 retains pad 2 firmly within the overall composite fabric of the diaper. By applying a transverse restraint on the flaps 43 and 44, sheet 5 also prevents the flaps from rolling outwardly. Sheet 5 is shown as having an aperture 46 in register with the aperture 40 in sheet 7. Since pad 2 is wrapped in the tissue wrap 30, the fibres and particles of super absorber are retained within the diaper fabric.

As stated above, the aperture 40 in sheet 7 is formed by partially cutting through the material of the sheet, which is preferably a spun bonded non-woven material, to leave approximately 2 to 5% of the depth of the material uncut. In the case of a sheet 7 made from a fibrous material, the desired depth of cut can readily be achieved by varying the pressure applied to the cutter blade used to form the cut so that the desired residual uncut fibres remain to hold the material within the line of the cut in position in the web of the material. This retains the integrity of sheet 7 so that it can be transported to be laminated with sheet 5 and the remainder of the diaper composite fabric without distortion. If the material within the line of the cut were removed, the sheet 7 could readily distort making handling and registration of sheet 7 with other components of the diaper fabric difficult. The material within the line of the cut can readily be removed by a suction pad or the like when sheet 7 is located in register with the underlying components of the diaper and before bonding of the components to one another takes place.

## Claims

1. A disposable sanitary article formed from a composite fabric comprising a fluid permeable next-to-the-skin layer, an outer fluid impermeable layer and an intermediate fluid absorbent layer or pad and having an axially elongated slit or aperture formed in a sheet to provide an apertured sheet in the next-to-the-skin layer of the composite fabric, the aperture or slit extending along or adjacent the centre line of the article, characterised in that:
a. the aperture or slit having opposed axial lips is formed in a single sheet of the composite diaper fabric within the plan area of the underlying absorbent pad or layer;
b. the opposed axial lips of the aperture or slit are provided with means for raising the distal edges of the lips with respect to the plane of the apertured sheet; and
c. the distal edges of the lips are caused to adopt an inwardly directed and upstanding configuration with respect to the aperture or slit so as to provide a pair of opposed inwardly directed upstanding axially extending barrier cuffs in the next-to-the-skin face of the article.

2. An article as claimed in claim 1, characterised in that the article is to be worn upon a person and extends over the crotch area of the person, and in that the aperture or slit is formed in the crotch area of the article.

3. An article as claimed in claim 2, characterised in that the article is a disposable diaper; and in that the axially elongated aperture or slit is formed in said single sheet along or adjacent the centre line of the crotch area of the diaper; and in that the opposed axial lips of the aperture or slit are provided with elastication or other means whereby the distal edges of the lips are caused to adopt an upstanding and inwardly directed configuration with respect to the said aperture or slit so as to provide said pair of opposed barrier cuff walls over at least the crotch length of the diaper.

4. An article as claimed in any one of the preceding claims, characterised in that the aperture or slit has a transverse dimension of at least 1 cm.

5. An article as claimed in any one of the preceding claims, characterised in that the aperture or slit extends for from 25 to 75% of the axial length of the article.

6. An article as claimed in any one of the preceding claims, characterised in that the means for raising the distal edges of the lips comprises elastication adjacent the distal edge of the lips.

7. An article as claimed in claim 6, characterised in that the elastication extends axially beyond the extremities of the aperture or slit.

8. An article as claimed in any one of the preceding claims, characterised in that a transverse cut is made in said first sheet at each end of the axial aperture or slit so as to form an axial flap of material along each axial edge of the aperture or slit, each of which flaps forms one of the barrier cuff walls; and in that the aperture or slit provides a generally rectangular aperture in said first sheet.

9. An article as claimed in claim 8, characterised in that an initial axial aperture or slit is formed which has a transverse dimension of from 40 to 90% of the desired transverse width of the aperture, and in that the distal edges of said flaps are folded downwardly and under the remainder of the material to extend the initial axial aperture or slit to its desired transverse dimension.

10. An article as claimed in any one of the preceding claims, characterised in that there is provided a secondary sheet of material intermediate the apertured sheet and the absorbent pad or layer.

11. An article as claimed in claim 10, characterised in that a third sheet is provided underlying said second sheet, and in that said third sheet carries particles of a super absorber material and is formed with an aperture therein which is at least in part in register with the aperture formed in said apertured sheet.

12. An article as claimed in any one of the preceding claims, characterised in that a recess is formed in said absorbent pad or layer, which recess is at least in part in register with the aperture formed in said apertured sheet.

13. An article as claimed in any one of the preceding claims, characterised in that the layers of the composite fabric are adhered or otherwise bonded together, except that the said apertured sheet is not bonded to the underlying sheets in the composite fabric over at least the plan area of the aperture or slit.

14. A method for forming a pair of barrier cuffs in a disposable sanitary article formed from a composite fabric comprising a fluid permeable next-to-the-skin layer, an outer fluid impermeable layer and an intermediate fluid absorbent layer or pad, characterised in that the method comprises:
a. forming an axially elongated slit or aperture in a single sheet in the next-to-the-skin layer of the fabric to provide an apertured sheet, the aperture or slit having axial opposed lips along each axial edge thereof, the aperture or slit extending along or adjacent the centre line of the article and being located within the plan area of the underlying absorbent pad or layer; and
b. providing the opposed axial lips of the aperture or slit with means for raising the distal edges of the lips with respect to the plane of the apertured sheet so as to provide a pair of opposed inwardly directed upstanding axially extending barrier cuffs in the next-to-the-skin layer of the article when the article is in its relaxed stated.

15. A method for forming a pair of barrier cuffs in a disposable sanitary article formed from a composite fabric comprising a fluid permeable next-to-the-skin layer, an outer fluid impermeable layer and an intermediate fluid absorbent layer or pad, by forming an axial aperture in a sheet in the next-to-the-skin surface layer of the article along or adjacent the centre line of the article by removing material from said sheet, characterised in that:
a. the next-to-the-skin sheet in which the aperture is formed is made from a non-woven fibrous material;
b. the fibrous material of the next-to-the-skin sheet is partially cut through along the periphery of the intended aperture, whereby the material of the sheet within the intended aperture is removably retained within the sheet; and
c. subsequently removing the material of the sheet within the intended aperture to form the aperture.
